# EUROPEAN PATENT SPECIFICATION

(11) **EP 2 704 670 B1**
(45) Date of publication and mention of the grant of the patent: **22.03.2017**
(21) Application number: 12722982.1
(22) Date of filing: 03.05.2012
(51) Int. Cl.: A61F 2/30, A61F 2/28

(54) **CRANIOTOMY PLUGS**
SCHÄDELKNOCHENDECKELS
BOUCHONS DE CRANIOTOMIE

(30) Priority: 03.05.2011 US 201161482039 P
(43) Date of publication of application: 12.03.2014
(73) Proprietor: Biodynamics, LLC, Hackensack, NJ 07601 (US)
(72) Inventor: RALPH, James, D., Bethlehem, PA 18017 (US); TROXELL, Thomas, N., Pottstown, PA 19465 (US); MICHELS, Mark, Glen Mills, PA 19342 (US)
(74) Representative: Beresford, Keith Denis Lewis
(86) International application number: PCT/US2012/036374
(87) International publication number: WO 2012/151432

(56) References cited:
- EP-A1- 1 808 140
- US-A- 4 745 914
- US-A- 5 503 164
- US-A- 5 634 944
- US-B1- 7 004 948

## Description

### FIELD OF THE INVENTION

The invention relates to cranial plugs used to fill small openings in the cranium or the burr holes made to facilitate cutting out a skull flap. In a further aspect, the invention relates to plugs which enhance bone growth and the consequent healing of the skull flap and the skull.

### BACKGROUND OF THE INVENTION

Surgical access to the brain for neurosurgical procedures is created by removing a portion of the patient's skull, a procedure termed a craniotomy. The craniotomy is determined by the location of the pathology within the brain, the safest/easiest access route and the degree of exposure required for the procedure. Once the location is determined, the first step is to create an initial perforation of the full thickness of the skull. Special skull perforators are available to create perfectly round holes but most surgeons simply use a rounded, end-cutting burr to create the perforation. Typically the perforation is in the range of about 11-15 millimeters (mm) in diameter. A surgeon may choose to create more than one perforation around the perimeter of the planned craniotomy. Some surgeons prefer a single perforation and others use more than one, but there is no standard number. Once this hole is created, it allows the insertion of a rotary powered surgical instrument (e.g., a craniotome) which is used to create a continuous cut (kerf) around the perimeter of the craniotomy. This kerf begins and ends at the perforation when there is one perforation or it runs from one perforation to another when more than one perforation is made in the skull. The kerf is made with a side cutting burr which is shielded from the dura (outer covering of the brain) by a foot plate on the craniotome. The foot plate extends below and forward of the cutting burr and the surgeon keeps the tip of the foot plate in contact with the inner surface of the skull as he performs the craniotomy. The typical kerf is made freehand with an approximately 2 mm diameter burr. The shape of the craniotomy is therefore highly variable and the kerf is not always oriented perpendicular to the skull. The kerf may be larger than 2 mm in some areas as well. Over the course of the kerf, the skull thickness will vary, typically over the range of 3-8 mm in adults.

Once the cut is complete, the skull flap is removed from the skull and placed on the sterile back table for reinsertion at the end of the procedure. After completion of the soft tissue surgery (typically 1-6 hours), the skull flap is inserted back into the craniotomy and fixated to prevent movement and restore the original contour of the skull. The surgeon may bias the skull flap toward one side or another to create bone-to-bone contact in a particular area or he may leave a gap around the entire flap. The scalp is then closed and the patient is sent to the neurosurgical intensive care unit for recovery.

If complications develop while the patient is in the hospital, there may be the need for emergency access to the brain through the craniotomy site. In addition, some patients may return for subsequent craniotomies in the same region, particularly in cases of recurrent tumors. Postoperative imaging studies (MRI or CT) are generally conducted on all patients. There is no clear evidence that the skull flap ever completely heals (solid bony union) in adults. It is more likely that a combination of new bone formation and fibrous connective tissue fills the gap between the skull and the skull flap.

From a surgeon's perspective, the method of reattaching the bone flap must be safe, simple to use, be rapidly applied, permit emergent re-entry, not interfere with postoperative imaging studies, provide stable fixation and have an acceptably low profile. The ideal method would result in complete fusion of the bone flap to the native skull with no long term evidence of prior surgery.

Current methods of reattaching the skull flap include drilling a series of small holes in the edge of the skull and the edge of the flap. Sutures are then passed through the corresponding holes and the flap is secured back into the skull opening from which it was taken. Because the fit is not exact due to the material removed by the craniotome, the flap can sag and sit slightly below the surface of the skull resulting in a depressed area that is obvious through the skin.

Another common reattachment method substitutes stainless steel wire for the suture material and fewer holes are used. There is still the risk of a cosmetically objectionable depressed area resulting. Metallic cranial fixation is (generally) only ever removed if it becomes symptomatic or if it interferes with subsequent surgeries.

More recently, surgeons have begun to use the titanium micro plates and screws that were developed for internal fixation of facial and finger bones. While this method results in a more stable and cosmetic result, it is relatively expensive, does not insure fusion and leaves foreign bodies at the surgical site.

All of these methods take ten minutes to one hour of additional surgery after the soft tissue (brain) surgery.

There is another method in which a titanium rivet (or clamp) is placed inside the skull with the stem of the rivet (clamp) passing between the skull and the flap. A large "pop rivet" type tool is used to force an upper titanium button down over the stem of the rivet, locking the flap and the skull in place between the upper and lower buttons. Three or four of these rivets and buttons are used to secure the flap in place. This method can be faster than other methods and less expensive than the titanium plates, but more expensive than sutures or wires. Just as with titanium plates and screws, fusion is not assured and foreign bodies remain in the patient.

By way of further background, reference is made to the following publications:
United States Patent US 5,503,164 (Friedman) describes a biocompatible scaffolding/cranial client with an insert segment positioned at the proximal surface of the bone defect and stabilised with support means, coupled with bone replacement material applied distal to the scaffolding insert segment and generally filling the defect. A kit of parts is provided, including tools for trimming bending or inserting the scaffolding material for the insert segment of the scaffolding or platform may be chosen from a wide variety of choices of material. Relatively rigid tabs may be connected to the insert segment for stabilising the insert segment in situ.
EP-A-1808140 discloses a cranial plug which is moulded into a predefined concave shape with flexible side walls.

### SUMMARY OF THE INVENTION

We have developed cranial plugs for burr holes or cranial perforations. The cranial plugs of the invention are secure and cosmetically acceptable. The plugs also can enhance bone growth in a manner which causes healing by means of bone-to-bone reattachment of the skull flap to the skull.

The present invention is directed to a cranial plug for a burr hole or a cranial perforation made in a skull during brain surgery, the cranial plug having a base portion and a plurality of flexible side walls extending out therefrom, wherein the cranial plug is substantially flat prior to insertion into the burr hole or cranial perforation, wherein the base portion is adapted to cover the dura so as to prevent access to a space between the dura matar and the inside surface of the skull and wherein the plurality of flexible side walls are: adapted to flex upwards from the base portion to form a concave shape when the base portion of the cranial plug is inserted into the burr hole or the cranial perforation; conform to the sidewalls of the burr hole or cranial perforation and act as spring fingers to hold the plug in place; and wherein the plurality of flexible side walls, when flexed upwards from the base portion, form a concave shaped containment space for receiving a bioactive substance to aid in fusion across the burr hole.

The cranial plugs of the invention are used to plug small circular openings in the skull such as those made by a surgeon to gain access for surgery or to insert a cutting instrument such as a craniotome to cut out a skull flap. Sometimes more than one small hole is made in the skull to facilitate cutting out a skull flap and, following surgery, the cranial plugs can be used to fill each of those holes, sometimes referred to as burr holes. The cranial plugs can be used by themselves, or in combination with fasteners, such as those described in U.S. Patent No. 8,080,042. The cranial plug design described herein also can be filled or partially filled with medication, bone paste, bone growth enhancers and the like.

The cranial plugs of the present invention do not contain a top flange-they are simply inserted into the burr hole, the sidewalls of the plug conform to the sidewalls of the burr hole and act as spring fingers to hold the plug in place and, in certain embodiments, provide openings through which the bone paste can move sideways to contact the bone. This also provides pathways for fusion.

### BRIEF DESCRIPTION OF THE DRAWINGS

The appended drawings are not intended to illustrate every embodiment of the invention but they are representative of embodiments within the principles of the invention. The drawings are for illustrative purposes and are not drawn to scale.
FIG. 1 is a perspective of a cranial plug.
FIG. 2 is a perspective view of a cranial plug.
FIG. 3 is a side view of a cranial plug.
FIG. 4 is bottom view of a cranial plug.
FIG. 5 is an alternate side view of a cranial plug.
FIG. 6 is a perspective top view of a cranial plug.
FIG. 7 is a top view of a cranial plug.
FIG. 8 is a perspective view of a cranial plug.
FIG. 9 is a view of an embodiment of a cranial plug of the present invention when inserted into a burr hole.
FIG. 10 is an embodiment of a cranial plug of the present invention when the plug is substantially flat prior to insertion into a burr hole.
FIG. 11 is a perspective view of a cranial plug.
FIG. 12 is a perspective view of a cranial plug.
FIG. 13 is a perspective view of a cranial plug.
FIG. 14 is an embodiment of a cranial plug of the present invention when the plug is substantially flat prior to insertion into a burr hole.

### DETAILED DESCRIPTION

The cranial plugs of the present invention may be made, in part or in its entirety, with bioabsorbable material. The term "bioabsorbable material" as used herein includes materials which are partially or completely bioabsorbable in the body.

Suitable bioabsorbable materials include collagen, polyglycolide, poly(lactic acid), copolymers of lactic acid and glycolic acid, poly-L-lactide, poly-L-lactate; crystalline plastics such as those disclosed in U.S. Pat. No. 6,632,503; bioabsorbable polymers, copolymers or polymer alloys that are self-reinforced and contain ceramic particles or reinforcement fibers such as those described in U.S. Pat. No. 6,406,498; bioresorbable polymers and blends thereof such as described in U.S. Pat. No. 6,583,232; copolymers of polyethylene glycol and polybutylene terephthalate, and the like. The foregoing list is not intended to be exhaustive. Other bioabsorbable materials can be used based upon the principles of the invention as set forth herein. Some of the most common include Poly-L-lactic acid (PLLA) Poly-DL-lactic acid (PDLLA) Polyglycolic acid (PGA) Polydioxanone (PDS) Polyorthoester (POE) Poly-C-capralactone (PCL).

Bioactive materials can be admixed with the bioabsorbable materials, impregnated in the bioabsorbable materials and/or coated on the outer surface thereof. Bioactive materials, including natural and/or synthetic materials, also can be used to fill cavities in the cranial plugs. These materials can include, for example, bioactive ceramic particles, bone chips or paste, platelet rich plasma (PRP), polymer chips, synthetic bone cement, autologous materials, allograft, cadaveric materials, xenograft, nanoparticles, nanoemulsions and other materials employing nanotechnology, capsules or reinforcement fibers. And they can contain, for example, antimicrobial fatty acids and related coating materials such as those described in Published U.S. Patent Publication No. 2004-0153125; antibiotics and antibacterial compositions; immunostimulating agents; tissue or bone growth enhancers and other active ingredients and pharmaceutical materials known in the art.

Some cranial plugs which are made with bioabsorbable material can be made by molding, extrusion, heat shrinking or coating the bioabsorbable material on a base which has been provided with attachment means such as those described in Published U.S. Patent Publication No. 2006-0142772. When the bioabsorbable material will have functional mechanical properties which are not made from the base material, the bioabsorbable material can be molded onto the base in the desired shape. Alternatively, the bioabsorbable material also can be coated, shrink wrapped or molded onto the base. If necessary, the bioabsorbable material can be machined to the desired shape and/or dimensions.

As will be apparent to those skilled the art, the sizes of the plugs can be varied to meet their intended applications. The shapes can take various forms in addition to those illustrated. And the sizes, lengths and widths can be varied for particular applications.

When the plug is inserted into the burr hole, at least a portion of the plug would cover the outer layer of the brain, the dura. The intent is to create a "floor" in the opening to aid in containment of autologous and other bioactive substances with the goal of creating fusion across the burr hole. Without this "floor", these substances could easily be pushed beneath the skull, into the space between the dura and inside of the skull. This would result in bioactive substances where they are not intended and it would also force the surgeon to use a larger volume of these materials than is necessary. The latter is a concern because bioactive substances are costly, or when harvested autologously, are available in limited quantity.

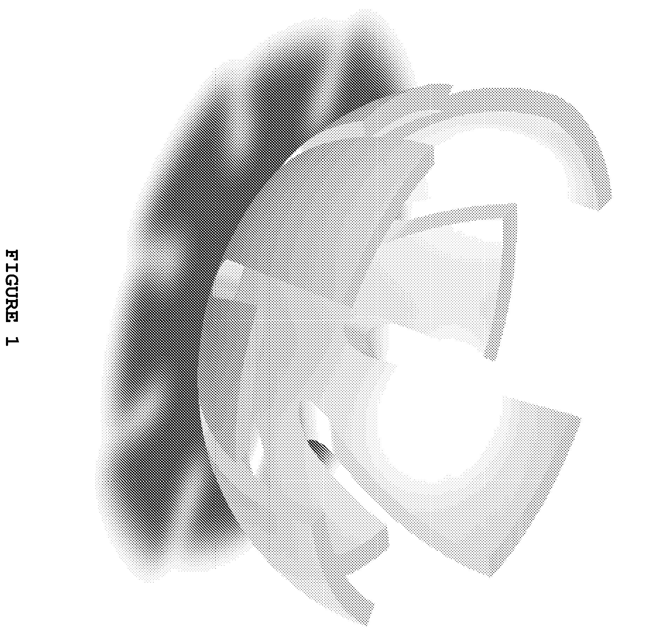

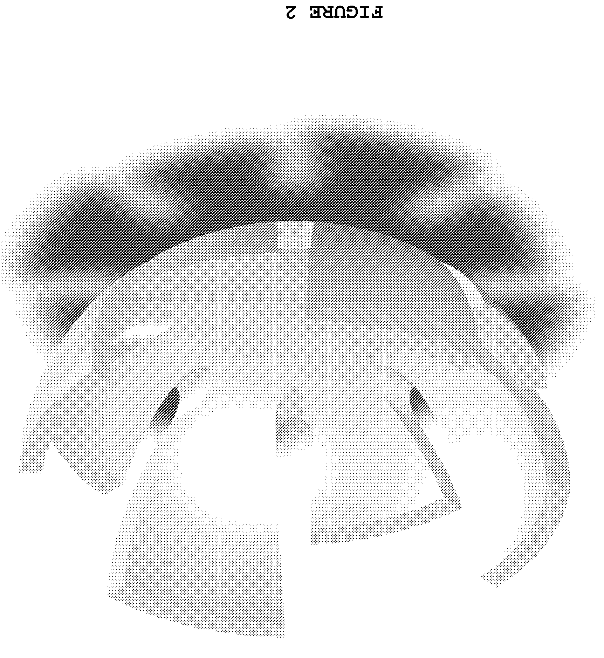

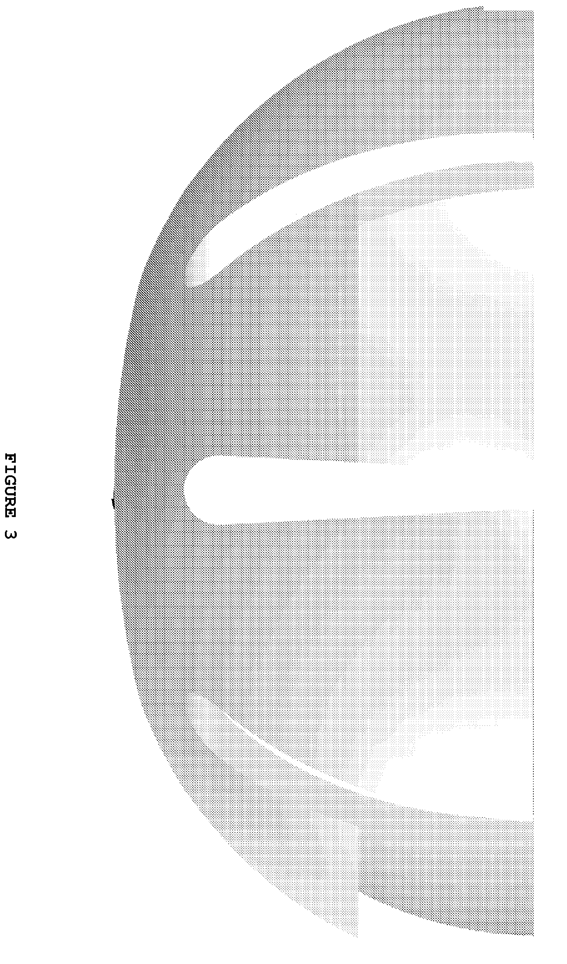

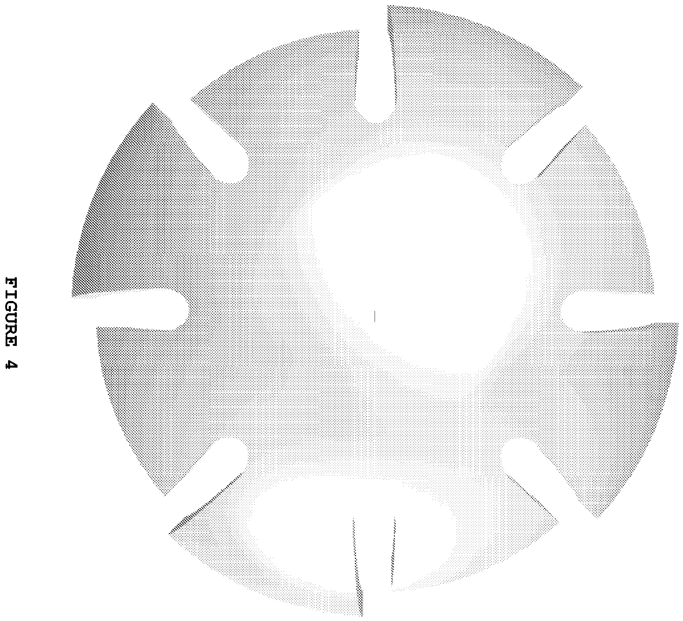

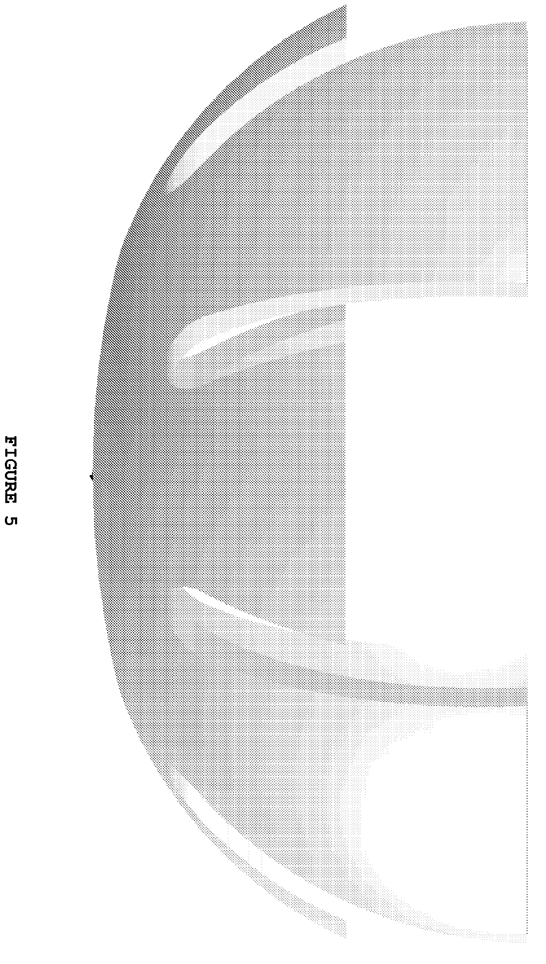

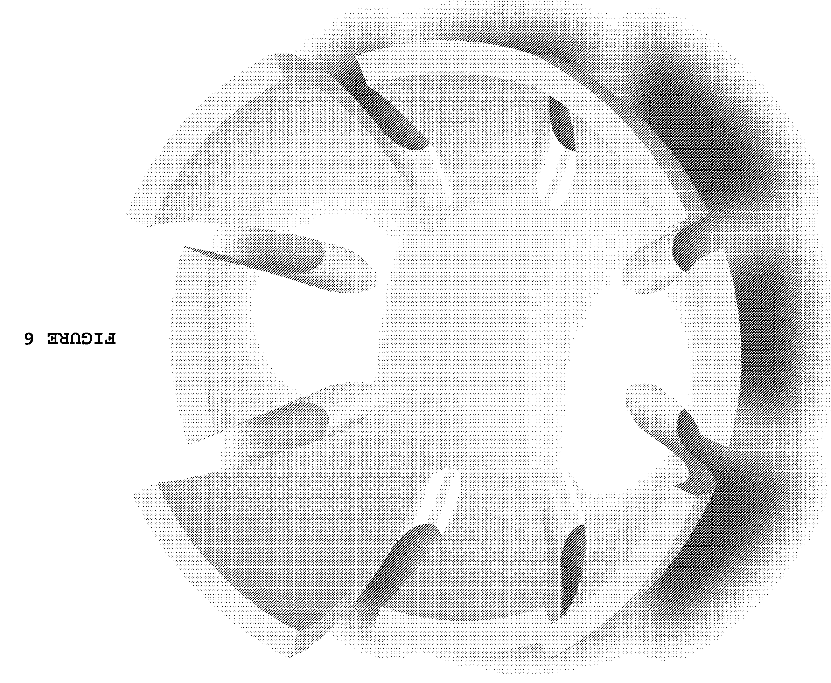

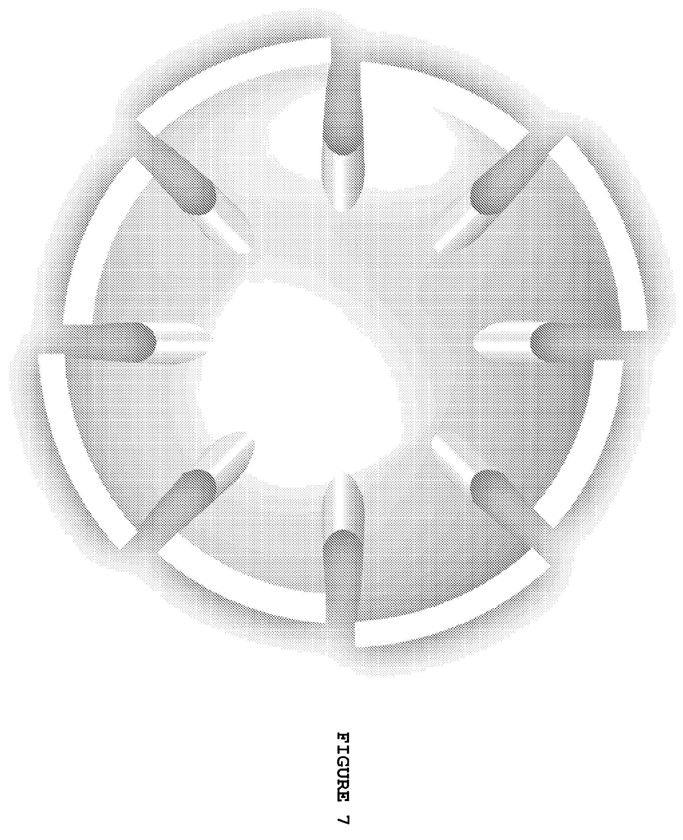

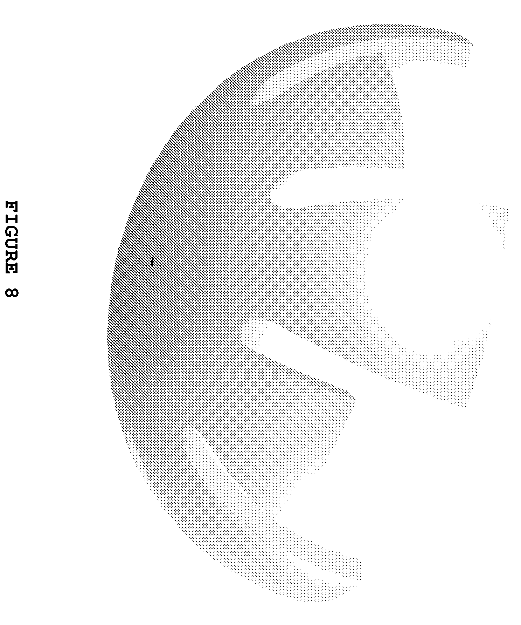

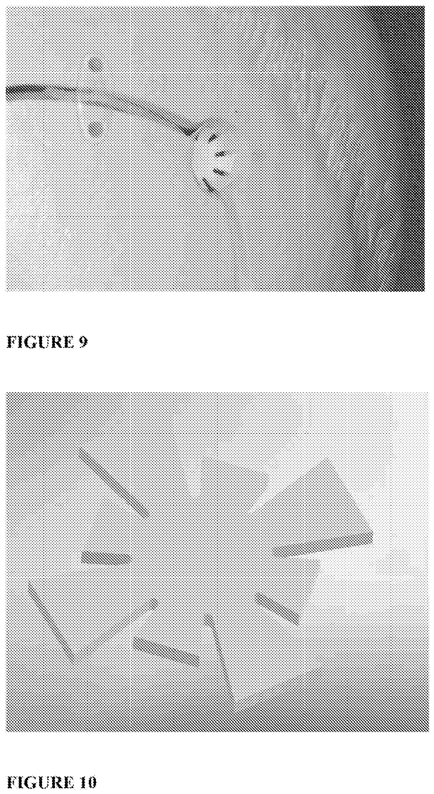

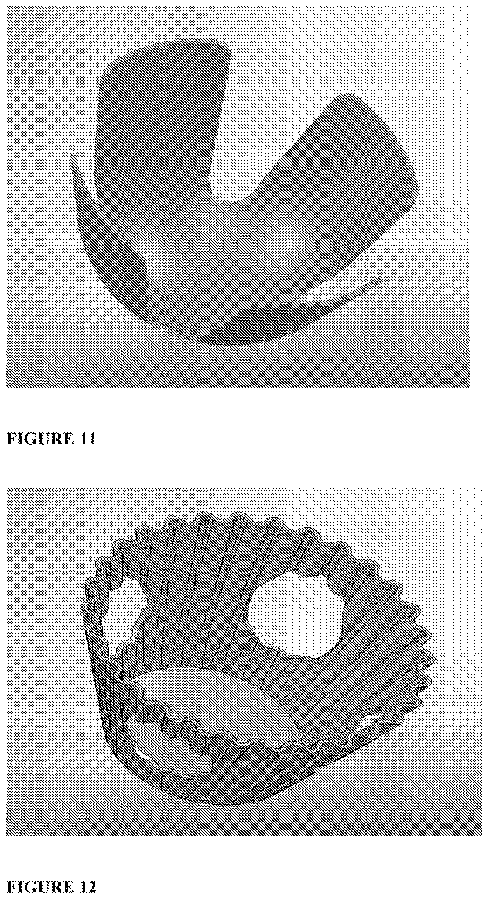

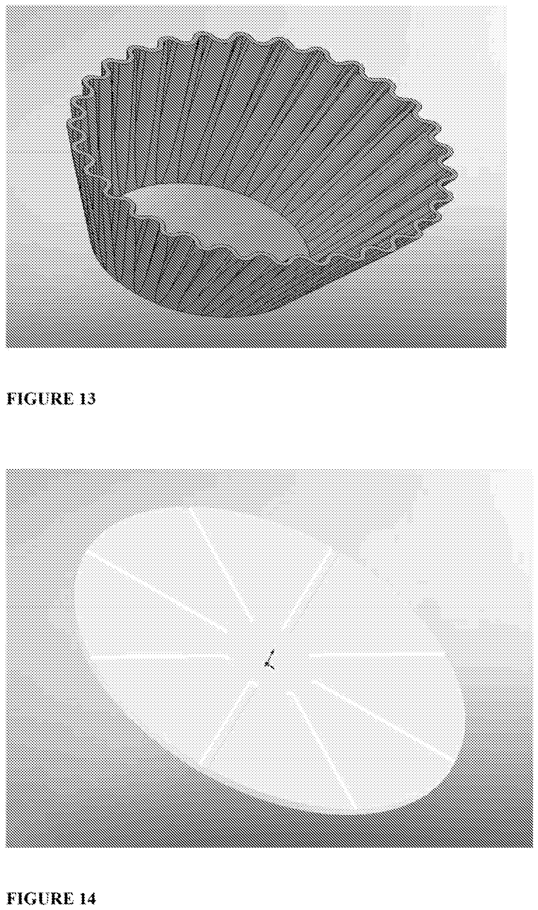

Any of the plugs disclosed in this application could be produced in either a preformed shape (e.g., Figures 1-8) which is not falling under the scope of the claimed invention or a flat condition (e.g., Figure 10). Either style would produce the desired result provided the material was sufficiently flexible. The installed plug may provide gaps, holes or slots in the sidewall to allow bone graft or bioactive materials to directly contact the sidewalls of the burr hole thereby improving fusion. Figures 12 and 13 show an embodiment with and without holes in a side wall.

In the flat condition, the plugs may be pre-scored or embossed to cause them to fold/collapse in a predetermined manner. Such an embodiment is shown in Figure 14.

In an alternate example (not shown), the cranial plug may be in essentially flat condition and tucked under the skull to cover the dura in the area(s) directly below the burr hole(s) before the skull flap is reattached. The plug/dura cover is made from a sheet of generally even thickness and can be in one piece or more than one piece. The cranial plug of this design would not be inserted into the burr hole and would not have any significant contact with the sidewalls of the burr hole.

## Claims

1. A cranial plug for a burr hole or a cranial perforation made in a skull during brain surgery, the cranial plug having a base portion and a plurality of flexible side walls extending out therefrom, wherein the cranial plug is substantially flat prior to insertion into the burr hole or cranial perforation, wherein the base portion is adapted to cover the dura so as to prevent access to a space between the dura matar and the inside surface of the skull and wherein the plurality of flexible side walls:
i) are adapted to flex upwards from the base portion to form a concave shape when the base portion of the cranial plug is inserted into the burr hole or the cranial perforation;
ii) conform to the sidewalls of the burr hole or cranial perforation and act as spring fingers to hold the plug in place; and
iii) when flexed upwards from the base portion, form said concave shape which is suitable for acting as a containment space for receiving a bioactive substance to aid in fusion across the burr hole.

2. The cranial plug of claim 1, wherein each of the plurality of flexible side walls have an elongated tabular shape.

3. The cranial plug of claim 2, wherein the plurality of flexible side walls are not in contact with each other.

4. The cranial plug of claim 2, wherein the plurality of flexible side walls have different dimensions.

5. The cranial plug of claim 2, wherein the plurality of flexible side walls have the same dimensions.

6. The cranial plug of claim 1, wherein the cranial plug comprises a bioabsorbable material.

7. The cranial plug of claim 6, wherein the bioabsorbable material is selected from the group consisting of collagen, polyglycolide, poly(lactic acid), copolymers of lactic acid, glycolic acid, poly-L-lactide, poly-L-lactate, copolymers of polyethylene glycol, polybutylene terephthalate and combinations thereof.

## Patentansprüche

1. Schädelknochendeckel für ein Bohrloch oder eine Schädelknochenperforation, das oder die während einer Gehirnoperation in einem Schädel gebildet wurde, wobei der Schädelknochendeckel einen Basisabschnitt und mehrere biegsame Seitenwände, die sich davon auswärts erstrecken, aufweist, wobei der Schädelknochendeckel vor dem Einsetzen in das Bohrloch oder die Schädelknochenperforation im Wesentlichen flach ist, wobei der Basisabschnitt dazu ausgelegt ist, die Hirnhaut so abzudecken, dass der Zugang zu einem Raum zwischen der Hirnhaut und der Innenfläche des Schädels verhindert wird, und wobei die mehreren biegsamen Seitenwände:
i) dazu ausgelegt sind, sich von dem Basisabschnitt aufwärts zu biegen, um eine konkave Form zu bilden, wenn der Basisabschnitt des Schädelknochendeckels in das Bohrloch oder die Schädelknochenperforation eingesetzt wird;
ii) sich an die Seitenwände des Bohrlochs oder der Schädelknochenperforation anzupassen und als Federfinger zu wirken, um den Deckel an seiner Stelle zu halten; und,
iii) wenn sie von dem Basisabschnitt aufwärts gebogen werden, die konkave Form zu bilden, die dazu geeignet ist, als Aufnahmeraum zur Aufnahme einer bioaktiven Substanz zur Unterstützung der Verschmelzung über das Bohrloch zu wirken.

2. Schädelknochendeckel nach Anspruch 1, wobei jede der mehreren biegsamen Seitenwände eine längliche tafelförmige Form aufweist.

3. Schädelknochendeckel nach Anspruch 2, wobei die mehreren biegsamen Seitenwände nicht miteinander in Kontakt stehen.

4. Schädelknochendeckel nach Anspruch 2, wobei die mehreren biegsamen Seitenwände unterschiedliche Abmessungen aufweisen.

5. Schädelknochendeckel nach Anspruch 2, wobei die mehreren biegsamen Seitenwände die gleichen Abmessungen aufweisen.

6. Schädelknochendeckel nach Anspruch 1, wobei der Schädelknochendeckel ein bioresorbierbares Material umfasst.

7. Schädelknochendeckel nach Anspruch 6, wobei das bioresorbierbare Material aus der Gruppe gewählt ist, die aus Kollagen, Polyglycolid, Poly(milchsäure), Copolymeren von Milchsäure, Glykolsäure, Poly-L-Lactid, Copolymeren von Polyethylengykol, Polybutylenterephthalat und Kombinationen davon besteht.

## Revendications

1. Bouchon crânien pour un trou de trépan ou une perforation crânienne formé dans un crâne avant une chirurgie cérébrale, le bouchon crânien ayant une partie de base et une pluralité de parois latérales flexibles s'étendant vers l'extérieur depuis celui-ci, le bouchon crânien étant sensiblement plat avant insertion dans le trou de trépan ou la perforation crânienne, la partie de base étant adaptée pour couvrir la dure-mère de manière à empêcher l'accès à un espace entre la dure-mère et la surface intérieure du crâne et dans lequel la pluralité de parois latérales flexibles :
i) sont adaptées pour fléchir vers le haut depuis la partie de base pour former une forme concave lorsque la partie de base du bouchon crânien est insérée dans le trou de trépan ou la perforation crânienne ;
ii) se conforment aux parois latérales du trou de trépan ou de la perforation crânienne et agissent comme des doigts à ressort pour maintenir le bouchon en place ; et
iii) lorsqu'elles sont fléchies vers le haut depuis la partie de base, forment ladite forme concave qui est adaptée pour agir en tant qu'espace de confinement pour recevoir une substance bioactive afin de faciliter la fusion de part et d'autre du trou de trépan.

2. Bouchon crânien de la revendication 1, dans lequel chacune de la pluralité de parois latérales flexibles a i une forme tabulaire allongée.

3. Bouchon crânien de la revendication 2, dans lequel la pluralité de parois latérales flexibles ne sont pas en contact les unes avec les autres.

4. Bouchon crânien de la revendication 2, dans lequel la pluralité de parois latérales flexibles ont différentes dimensions.

5. Bouchon crânien de la revendication 2, dans lequel la pluralité de parois latérales flexibles ont les mêmes dimensions.

6. Bouchon crânien de la revendication 1, le bouchon crânien comprenant un matériau biorésorbable.

7. Bouchon crânien de la revendication 6, dans lequel le matériau biorésorbable est choisi dans le groupe constitué des collagène, polyglycolide, poly(acide lactique), copolymères d'acide lactique, acide glycolique, poly-L-lactide, poly-L-lactate, copolymères de polyéthylène glycol, téréphtalate de polybutylène et des combinaisons de ceux-ci.
